# EUROPÄISCHE PATENTSCHRIFT

(11) **EP 3 173 402 B1**
(45) Veröffentlichungstag und Bekanntmachung des Hinweises auf die Patenterteilung: **21.03.2018**
(21) Anmeldenummer: 15196938.3
(22) Anmeldetag: 30.11.2015
(51) Int. Cl.: C07C 253/10, C07C 253/34, C07C 255/46

(54) **FEINREINIGUNG VON ISOPHORONNITRIL DURCH SCHMELZKRISTALLISATION**
FINE PURIFICATION OF ISOPHORONE NITRILE BY MELT CRYSTALLIZATION
ÉPURATION D'ISOPHORONE-NITRILE PAR CRISTALLISATION DE FUSION

(43) Veröffentlichungstag der Anmeldung: 31.05.2017
(73) Patentinhaber: Evonik Degussa GmbH, 45128 Essen (DE)
(72) Erfinder: NITZ, Jörg-Joachim, 45257 Essen (DE); KOHLSTRUK, Stephan, 45966 Gladbeck (DE); JANSEN, Robert, 46244 Bottrop (DE); MÜLLER, Anja, 44135 Dortmund (DE); CASSENS, Jan, 45665 Recklinghausen (DE); HENGSTERMANN, Axel, 48308 Senden (DE)

(56) Entgegenhaltungen:
- EP-A1- 0 616 998
- EP-A2- 0 792 867
- WO-A2-01/90066
- CN-A- 101 851 178
- CN-A- 102 199 109
- US-A- 5 011 968
- Jansens, P.J. et al.: "Melt Crystallization", , 2000, Seiten 966-975, XP55269551, Gefunden im Internet: URL:https://www.thevespiary.org/library/Fi les_Uploaded_by_Users/Sedit/Chemical Analysis/Encyclopedia of Separation Science/Level II - Methods and Instrumentation/CRYSTALLIZATION/Melt Crystallization.pdf [gefunden am 2016-04-29]

## Beschreibung

Die vorliegende Erfindung betrifft die Feinreinigung von Isophoronnitril (IPN) durch Schmelzkristallisation.

Die Basen katalysierte Umsetzung von Blausäure (HCN) mit alpha, beta-ungesättigten cyclischen (oder acyclischen) Ketonen ist eine bekannte Reaktion (Hydrocyanation of Conjugated Carbonyl Compounds, CHAPTER 3, Wataru Nagata and Mitsuru Yoshioka, Wiley 2005).

Aus der EP 2 721 002 sind zahlreiche Verfahren zur Herstellung von Isophoronnitril bekannt, siehe die dort zitierten Dokumente.

Isophoronnitril (IPN) entsteht durch die Reaktion von Isophoron (IP) mit Blausäure unter Zuhilfenahme eines Katalysators. Vorzugsweise wird eine homogene Basenkatalyse eingesetzt, hierzu werden Alkalialkoholate, insbesondere Natriummethanolat, als Katalysator eingesetzt.

US 5 011 968 A offenbart ein Verfahren zur Herstellung von Isophoronnitril, bei dem Isophoron mit Cyanwasserstoff in Gegenwart eines quartären Ammoniumhydroxidkatalysators umgesetzt wird. Dabei werden die Katalysatorrückstände durch Durchleiten eines erhitzten Inertgases und das nicht umgesetzte Cyanid durch Ansäuern und Durchleiten eines erhitzten Inertgases eliminiert.

CN 102 199 109 A offenbart ein Verfahren zur Herstellung von Isophoronnitril, bei dem als Katalysator ein basisches Anionenaustauscherharz verwendet wird.

CN 101 851 178 A offenbart ein Verfahren zur Herstellung von Isophoronnitril, bei dem Isophoron und Cyanwasserstoff in Gegenwart eines basischen Katalysators umgesetzt, anschließend mit einer organischen Säure neutralisiert und mit einem Stabilisator und mit einem ggf. wässrigen Lösemittel versetzt wird.

Isophoronnitril wird durch die Umsetzung von Cyanwasserstoff (HCN) und Isophoron (IP) in Gegenwart von Natriummethanolat als Katalysator hergestellt, wie in der EP 2649043 beschrieben. Das bei dieser Reaktion entstehende Produktgemisch enthält neben dem Produkt Isophonnitril, die nicht umgesetzten Edukte Cyanwasserstoff und Isophoron sowie den Katalysator, Methanol und Rückstände. Dieses Produktgemisch wird in einem mehrstufigen Prozess durch Destillation aufgereinigt, wobei das Isophoronnitril in der letzten Stufe rein erhalten wird. Dabei können Reinheiten von mindestens 99,8 Gew.-% erreicht werden. Dieses aufgereinigte Isophoronnitril wird in einer weiteren Reaktion zu Isophorondiamin umgesetzt. Die noch enthaltenen geringen Mengen an Verunreinigungen im aufgereinigten Isophoronnitril können aber die Lebensdauer des bei der Isophorondiaminherstellung verwendeten Katalysators beeinträchtigen und die Farbstabilität des Isophorondiamins herabsetzen.

Aufgabe war es, die Aufarbeitung des Produktgemischs, bestehend aus Isophonnitril, den nicht umgesetzten Edukten Cyanwasserstoff und Isophoron sowie dem Katalysator, Methanol und Rückständen zu verbessern.

Überraschenderweise wurde gefunden, dass mit einer an die Destillation angeschlossenen Schmelzkristallisation die Reinheit des Isophoronnitrils gesteigert werden kann und so störende Verunreinigungen entfernt werden können.

Gegenstand der Erfindung ist ein Verfahren zur Aufreinigung von Isophoronnitril aus der Herstellung von Isophoronnitril aus Isophoron und Blausäure in Gegenwart eines Katalysators, dadurch gekennzeichnet, dass die Abtrennung von Verunreinigungen im Isophoronnitril durch eine Schmelzkristallisation erfolgt.

Die Aufarbeitung des Produktgemischs, im Wesentlichen bestehend aus Isophonnitril, den nicht umgesetzten Edukten Cyanwasserstoff und Isophoron sowie dem Katalysator, Methanol und Rückständen, erfolgt erfindungsgemäß durch Schmelzkristallisation, bei der Verunreinigungen im Isophoronnitril entfernt werden, die nicht in der Destillation entfernt werden konnten.

Bei der verfahrenstechnischen Umsetzung besteht die Möglichkeit, die Kristallisation des Isophoronnitrils als Suspensionskristallisation oder als Schichtkristallisation durchzuführen.

Insbesondere die Schichtkristallisation bietet sich für das vorliegende Trennproblem an. Die Schichtkristallisation kann dabei sowohl im statischen als auch im dynamischen Betrieb durchgeführt werden, wobei eine Kombination aus beiden Prozessschritten bevorzugt wird.

Nach Ende der Kristallisation wird die Mutterlauge abgelassen und ein Schwitzprozess eingeleitet. Dieser kann zwischen 1 Minute bis zu 20 Minuten dauern. Nach Ablassen der Schwitzfraktion kann das Kristallisat abgeschmolzen und weiter prozessiert werden. Die Kristallisation findet in einer oder bevorzugt in mehreren Stufen statt, wobei bei der mehrstufigen Variante die abgelassene Mutterlauge und die Schwitzfraktion sowie das Kristallisat in unterschiedlichen Pufferbehältern gesammelt werden. Je nach gewünschter Reinheit wird das Kristallisat erneut prozessiert. Zur Erhöhung der Ausbeute wird die Mutterlauge sowie die Schwitzfraktion erneut prozessiert.

Ein bevorzugter Apparateaufbau einer dynamischen Kristallisation ist der Fallfilmkristallisator, bei dem der Umlauf des aufzureinigenden Produktgemischs, an der Wandung eines von außen gekühlten Rohres herablaufen gelassen wird und dabei an der Rohrwandung zu kristallisieren beginnt. Die Länge des Rohres kann dabei von 2m bis zu 16 m betragen. Zudem können für höhere Durchsätze mehrere Rohre als Rohrbündel verwendet werden, wobei über einen Verteiler in jedem der Rohre für eine gleichmäßige Auftragung des Filmes, bestehend aus dem aufzureinigenden Produktgemisch, gesorgt wird.

Eine statische Kristallisation kann im Fallfilmkristallisator, auch ohne Umlauf, gefahren werden, wird jedoch bevorzugt in einem Plattenkristallisator durchgeführt.

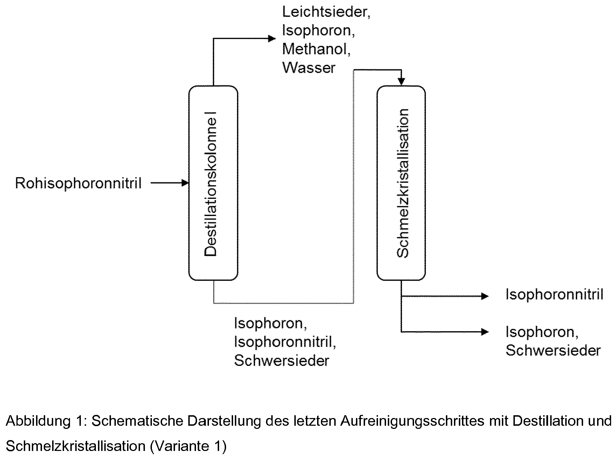

Beschreibung der bevorzugten Ausführungsform zur Aufreinigung von Isophoronnitril mittels Schmelzkristallisation (Variante 1)
Zunächst wird das Produktgemisch, bestehend aus Isophonnitril, den nicht umgesetzten Edukten Cyanwasserstoff und Isophoron sowie dem Katalysator, Methanol und Rückständen durch Destillation in einer ersten Kolonne aufgearbeitet, wobei Wasser, Leichtsieder und Methanol vollständig und mehr als 85% des Isophoron abgetrennt werden. In einer ersten Variante wird eine Destillation gleichzeitig zur Abtrennung des Wassers, wobei das ebenfalls abgetrennte Isophoron mittels eines Partialkondensators auskondensiert wird und in die Reaktion zurückgeführt wird. Das aus dem Sumpf austretende IPN/IP Gemisch wird anschließend in die Apparatur der Schmelzkristallisation geleitet (Abbildung 1). Anschließend wird nach Ende der Kristallisation wie oben beschrieben, verfahren.

Gegenstand der Erfindung ist somit auch ein Verfahren zur Aufreinigung von Isophoronnitril durch Schmelzkristallisation, wobei das Produktgemisch im Wesentlichen aus Isophoronnitril, den nicht umgesetzten Edukten Cyanwasserstoff und Isophoron sowie dem Katalysator, Methanol und Rückständen wie folgt aufgereinigt wird:
I. Aufreinigung des Produktgemischs durch eine Destillation, durch
   1. Abtrennung von Wasser, Leichtsiedern und Methanol sowie mindestens 85 % des Isophoron am Kopf der Kolonne, wobei der Isophoron-Anteil mittels eines Partialkondensators kondensiert und in die Reaktion zurückgeführt wird,
   2. Abtrennung im Sumpf der Kolonne von Isophoronnitril, und der Restmenge von Isophoron und Verunreinigungen (Schwersieder);
II. Das im Sumpf anfallende Gemisch aus I.2. wird einer Schmelzkristallisation zugeführt, wobei die Verunreinigungen abgetrennt werden und das Isophonnitril isoliert wird.

Beschreibung der bevorzugten Ausführungsform zur Aufreinigung von Isophoronnitril mittels Schmelzkristallisation (Variante 2)

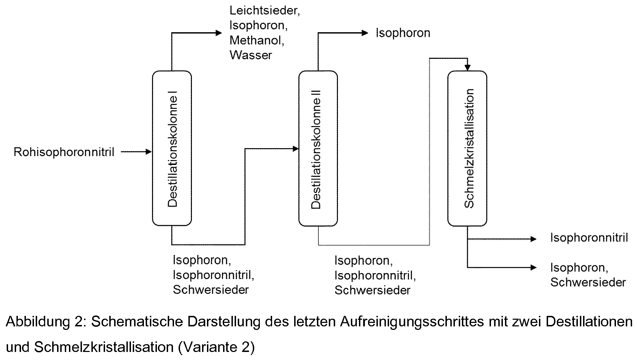

Zunächst wird das Produktgemisch, bestehend aus Isophonnitril, den nicht umgesetzten Edukten Cyanwasserstoff und Isophoron sowie dem Katalysator, Methanol und Rückständen durch Destillation in einer ersten Kolonne aufgearbeitet, wobei Wasser, Leichtsieder, Methanol und teiweise das Isophoron, bevorzugt maximal 10 %, abgetrennt werden. Danach folgt eine weitere Destillation des aus dem Sumpf der ersten Kolonne erhaltenen Isophoronnitrils, verunreinigt mit Isophoron und Schwersiedern. Die Destillation in der zweiten Kolonne wird dabei zur Abtrennung von reinem Isophoron am Kopf der Kolonne verwendet, wobei das Isophoronnitril, verunreinigt mit Resten von Isophoron (max. 15 Gew.%) und Schwersiedern im Sumpf der Kolonne abgenommen wird und einer Schmelzkristallisation zugeführt wird (Abbildung 2). Anschließend wird nach Ende der Kristallisation wie oben beschrieben, verfahren.

Gegenstand der Erfindung ist somit auch ein Verfahren zur Aufreinigung von Isophoronnitril durch Schmelzkristallisation, wobei der Produktstrom aus dem Reaktionsreaktor zur Herstellung von Isophoronnitril im Wesentlichen aus Isophoron, Blausäure und Katalysator, Rohisophoronnitril, wie folgt aufgereinigt wird:
I. Aufreinigung des Produktstroms durch eine erste Destillation
   a) wobei Wasser, Leichtsieder, Methanol und teilweise das Isophoron, bevorzugt maximal 10 %, am Kopf der Kolonne abgetrennt werden, und
   b) Abtrennung im Sumpf der Kolonne von Isophoronnitril, und der Restmenge von Isophoron und Verunreinigungen (Schwersieder);
II. Aufreinigung des Produktstroms aus I. b) in einer zweiten Destillationskolonne und Abtrennung des Isophoronnitrils, Isophorons und Verunreinigungen aus dem Sumpf der Kolonne;
III. Das im Sumpf anfallende Gemisch aus II. wird einer Schmelzkristallisation zugeführt, wobei die Verunreinigungen abgetrennt werden und das Isophonnitril isoliert wird.

### Beispiele

Zur Untersuchung dieses Aufreinigungsverfahrens wurde ein Fallfilmkristallisator mit einem Fallrohr von 2 m Länge verwendet. Das in den Kristallisator überführte Rohprodukt wird in einem senkrecht angeordneten, von außen gekühlten Fallrohr an der Rohrwandung ausgefroren. Die in der Mutterlauge verbleibenden Verunreinigungen werden im Anschluss abgelassen und das Kristallisat als Reinprodukt abgeschmolzen und abgelassen. Als weiterer Aufreinigungsschritt wurde nach Kristallisation ein Schwitzvorgang eingeleitet.

Als Medium wurde Rohisophoronnitril dem existierenden Prozess vor dem letzten Aufreinigungsschritt entnommen. Damit wurde eine mit Rückständen verunreinigte Isophoronnitrilmischung verwendet. Diese Mischung wurde zum einen rein und zum anderen mit künstlich zugesetzten Isophoron (Gesamtisophorongehalt 12,5 Gew.-%) verwendet.

### Beispiel 1 - Variante 1

Ein Rohisophoronnitril wurde als Feed bei statischer Betriebsweise kristallisiert (Kühlmedium bis 40°C), wobei der Kristallisationsraum mit Feed gefüllt und im Anschluss eine Kristallisation aus der im Fallrohr stehenden Mutterlauge durchgeführt wurde. Nach Ablassen der Mutterlauge wurde ein Schwitzprozess bei 67 °C eingeleitet, um weitere Verunreinigungen aus dem Kristallisat zu entfernen. Die Fraktionen wurden jeweils beprobt und deren Isophoronnitrilanteil bestimmt. Die Ergebnisse sind Tabelle 1 zu entnehmen, hier ist erkennbar, dass sich die Isophoronnitrilreinheit von 98.2 auf 99.3 Gew.-% steigern lässt.

**Tabelle 1: Isophoronnitril-Konzentrationen nach statischer Kristallisation in den einzelnen Fraktionen**

| | | |
|---|---|---|
| Feedkonzentration | 98.2 | Gew.-% |
| Konzentration in Mutterlauge | 97.2 | Gew.-% |
| Konzentration in Kristallisat | 99.3 | Gew.-% |

### Beispiel 2 - Variante 2

Es wurde die Aufreinigung einer Feedlösung aus Rohisophoronnitril mit einem IP Anteil von 13,2 Gew.-% untersucht. Für die Kristallisation wurde der Kristallisator dynamisch betrieben, wobei die Mutterlauge im ständigen Umlauf aus dem Sumpf des Kristallisators über eine Steigleitung in den Kopf des Kristallisators rückgeführt wurde. Das Kristallisat wurde dabei aus der über dem Fallrohr als Film zurücklaufenden Mutterlauge (Kühlmedium bis 40°C) ausgefroren. Nach Ablassen der Mutterlauge wurde ein Schwitzprozess bei 67 °C eingeleitet, um weitere Verunreinigungen aus dem Kristallisat zu entfernen. Die Fraktionen wurden jeweils beprobt und deren Isophoronnitrilanteil bestimmt. Die Ergebnisse sind Tabelle 2 zu entnehmen. Auch hier konnte eine Verbesserung der Reinheit erzielt werden, womit auch gezeigt werden konnte, dass Isophoronnitril selektiv von Isophoron mit Hilfe der Kristallisation getrennt werden kann.

**Tabelle 2: Isophoronnitril Konzentrationen nach dynamischer Kristallisation in den einzelnen Fraktionen**

| | | |
|---|---|---|
| Feedkonzentration | 85.9 | Gew.-% |
| Konzentration in Mutterlauge | 83.1 | Gew.-% |
| Konzentration in Kristallisat | 93.8 | Gew.-% |

## Patentansprüche

1. Verfahren zur Aufreinigung von Isophoronnitril aus der Herstellung von Isophoronnitril aus Isophoron und Blausäure in Gegenwart eines Katalysators, **dadurch gekennzeichnet, dass** die Abtrennung von Verunreinigungen im Isophoronnitril durch Schmelzkristallisation erfolgt.

2. Verfahren zur Aufreinigung von Isophoronnitril nach Anspruch 1, wobei die Schmelzkristallisation durch Suspensionskristallisation oder durch Schichtkristallisation erfolgt.

3. Verfahren zur Aufreinigung von Isophoronnitril nach mindestens einem der vorherigen Ansprüche, wobei die Schmelzkristallisation als Schichtkristallisation im statischen Betrieb oder im dynamischen Betrieb, oder einer Kombination aus beiden Prozessschritten durchgeführt wird.

4. Verfahren zur Aufreinigung von Isophoronnitril nach mindestens einem der vorherigen Ansprüche, wobei die Schmelzkristallisation in einer Stufe oder in mehreren Stufen durchgeführt wird.

5. Verfahren zur Aufreinigung von Isophoronnitril nach mindestens einem der vorherigen Ansprüche, wobei die Schmelzkristallisation in mehreren Stufen durchgeführt wird, und wobei die abgelassene Mutterlauge und die Schwitzfraktion sowie das Kristallisat in unterschiedlichen Pufferbehältern gesammelt werden.

6. Verfahren zur Aufreinigung von Isophoronnitril nach mindestens einem der vorherigen Ansprüche, wobei die dynamische Schmelzkristallisation in mindestens einem Fallfilmkristallisator durchgeführt wird.

7. Verfahren zur Aufreinigung von Isophoronnitril nach mindestens einem der vorherigen Ansprüche, wobei die statische Schmelzkristallisation in mindestens einem Fallfilmkristallisator oder in einem mindestens einem Plattenkristallisator durchgeführt wird.

8. Verfahren zur Aufreinigung von Isophoronnitril durch Schmelzkristallisation nach mindestens einem der vorherigen Ansprüche, wobei das Produktgemisch im Wesentlichen aus Isophoronnitril, den nicht umgesetzten Edukten Cyanwasserstoff und Isophoron sowie dem Katalysator, Methanol und Rückständen wie folgt aufgereinigt wird:
I. Aufreinigung des Produktgemischs durch eine Destillation, durch
1. Abtrennung von Wasser, Leichtsiedern und Methanol sowie mindestens 85 % des Isophoron am Kopf der Kolonne, wobei der Isophoron-Anteil mittels eines Partialkondensators kondensiert und in die Reaktion zurückgeführt wird,
2. Abtrennung im Sumpf der Kolonne von Isophoronnitril, und der Restmenge von Isophoron und Verunreinigungen (Schwersieder);
II. Das im Sumpf anfallende Gemisch aus I.2. wird einer Schmelzkristallisation zugeführt, wobei die Verunreinigungen abgetrennt werden und das Isophonnitril isoliert wird.

9. Verfahren zur Aufreinigung von Isophoronnitril durch Schmelzkristallisation nach mindestens einem der Ansprüche 1-7, wobei der Produktstrom aus dem Reaktionsreaktor zur Herstellung von Isophoronnitril im Wesentlichen aus Isophoron, Blausäure und Katalysator, Rohisophoronnitril, wie folgt aufgereinigt wird:
I. Aufreinigung des Produktstroms durch eine erste Destillation
a) wobei Wasser, Leichtsieder, Methanol und teilweise das Isophoron, bevorzugt maximal 10 %, am Kopf der Kolonne abgetrennt werden,
und
b) Abtrennung im Sumpf der Kolonne von Isophoronnitril, und der Restmenge von Isophoron und Verunreinigungen (Schwersieder);
II. Aufreinigung des Produktstroms aus I. b) in einer zweiten Destillationskolonne und Abtrennung des Isophoronnitrils, Isophorons und Verunreinigungen aus dem Sumpf der Kolonne;
III. Das im Sumpf anfallende Gemisch aus II. wird einer Schmelzkristallisation zugeführt, wobei die Verunreinigungen abgetrennt werden und das Isophonnitril isoliert wird.

## Claims

1. Process for purifying isophoronenitrile from the production of isophoronenitrile from isophorone and hydrocyanic acid in the presence of a catalyst, **characterized in that**
the removal of impurities in the isophoronenitrile is effected by melt crystallization.

2. Process for purifying isophoronenitrile according to Claim 1, wherein the melt crystallization is effected by suspension crystallization or by layer crystallization.

3. Process for purifying isophoronenitrile according to at least one of the preceding claims, wherein the melt crystallization is carried out as a layer crystallization in static operation or in dynamic operation or a combination of both process steps.

4. Process for purifying isophoronenitrile according to at least one of the preceding claims, wherein the melt crystallization is carried out in one stage or in a plurality of stages.

5. Process for purifying isophoronenitrile according to at least one of the preceding claims, wherein the melt crystallization is carried out in a plurality of stages and wherein the discharged mother liquor and the sweated fraction and also the crystallizate are collected in different buffering containers.

6. Process for purifying isophoronenitrile according to at least one of the preceding claims, wherein the dynamic melt crystallization is carried out in at least one falling film crystallizer.

7. Process for purifying isophoronenitrile according to at least one of the preceding claims, wherein the static melt crystallization is carried out in at least one falling film crystallizer or in an at least one plate crystallizer.

8. Process for purifying isophoronenitrile by melt crystallization according to at least one of the preceding claims, wherein the product mixture composed essentially of isophoronenitrile, the unconverted reactants hydrogen cyanide and isophorone and also the catalyst, methanol and residues is purified as follows:
I. purification of the product mixture by a distillation by
1. removal of water, low boilers and methanol and also at least 85% of the isophorone at the top of the column, the isophorone proportion being condensed by means of a partial condenser and recycled into the reaction,
2. removal in the bottom of the column of isophoronenitrile and the remaining isophorone and impurities (high boilers);
II. the mixture from 1.2. accumulating in the column-bottom is sent to a melt crystallization to remove the impurities and isolate the isophoronenitrile.

9. Process for purifying isophoronenitrile by melt crystallization according to at least one of Claims 1-7, wherein the product stream from the reaction reactor for producing isophoronenitrile composed essentially of isophorone, hydrocyanic acid and catalyst, crude isophoronenitrile, is purified as follows:
I. purification of the product stream by a first distillation
a) to remove water, low boilers, methanol and some, preferably not more than 10%, of the isophorone at the top of the column,
and
b) removal in the bottom of the column of isophoronenitrile and the remaining isophorone and impurities (high boilers);
II. purification of the product stream from I. b) in a second distillation column and removal of the isophoronenitrile, isophorone and impurities from the bottom of the column;
III. the mixture from II accumulating in the column-bottom is sent to a melt crystallization to remove the impurities and isolate the isophoronenitrile.

## Revendications

1. Procédé de purification d'isophorone-nitrile issu de la fabrication d'isophorone-nitrile à partir d'isophorone et d'acide cyanhydrique en présence d'un catalyseur, **caractérisé en ce que** la séparation d'impuretés dans l'isophorone-nitrile a lieu par cristallisation à l'état fondu.

2. Procédé de purification d'isophorone-nitrile selon la revendication 1, dans lequel la cristallisation à l'état fondu a lieu par cristallisation en suspension ou par cristallisation en couches.

3. Procédé de purification d'isophorone-nitrile selon au moins l'une quelconque des revendications précédentes, dans lequel la cristallisation à l'état fondu est réalisée sous la forme d'une cristallisation en couches en mode statique ou en mode dynamique, ou d'une combinaison des deux étapes de procédé.

4. Procédé de purification d'isophorone-nitrile selon au moins l'une quelconque des revendications précédentes, dans lequel la cristallisation à l'état fondu est réalisée en une étape ou en plusieurs étapes.

5. Procédé de purification d'isophorone-nitrile selon au moins l'une quelconque des revendications précédentes, dans lequel la cristallisation à l'état fondu est réalisée en plusieurs étapes, et dans lequel la liqueur mère déchargée et la fraction ressuée, ainsi que le cristallisat sont recueillis dans des contenants tampons différents.

6. Procédé de purification d'isophorone-nitrile selon au moins l'une quelconque des revendications précédentes, dans lequel la cristallisation à l'état fondu dynamique est réalisée dans au moins un cristallisateur à film tombant.

7. Procédé de purification d'isophorone-nitrile selon au moins l'une quelconque des revendications précédentes, dans lequel la cristallisation à l'état fondu statique est réalisée dans au moins un cristallisateur à film tombant ou dans au moins un cristallisateur à plaques.

8. Procédé de purification d'isophorone-nitrile par cristallisation à l'état fondu selon au moins l'une quelconque des revendications précédentes, dans lequel le mélange de produits essentiellement constitué par l'isophorone-nitrile, les réactifs cyanure d'hydrogène et isophorone non réagis, ainsi que le catalyseur, du méthanol et des résidus, est purifié de la manière suivante :
I. le mélange de produits est purifié par une distillation, par
1. séparation de l'eau, des composants de point d'ébullition faible et du méthanol, ainsi que d'au moins 85 % de l'isophorone à la tête de la colonne, la fraction d'isophorone étant condensée au moyen d'un condensateur partiel et recyclée dans la réaction,
2. séparation dans le fond de la colonne de l'isophorone-nitrile, et de la quantité résiduelle d'isophorone et d'impuretés (composants de point d'ébullition élevé) ;
II. le mélange de 1.2 formé dans le fond est introduit dans une cristallisation à l'état fondu, les impuretés étant séparées et l'isophorone-nitrile étant isolé.

9. Procédé de purification d'isophorone-nitrile par cristallisation à l'état fondu selon au moins l'une quelconque des revendications 1 à 7, dans lequel le courant de produits issu du réacteur de réaction pour la fabrication d'isophorone-nitrile essentiellement constitué par de l'isophorone, de l'acide cyanhydrique et le catalyseur, de l'isophorone-nitrile brut, est purifié de la manière suivante :
I. le courant de produits est purifié par une première distillation,
a) l'eau, les composants de point d'ébullition faible, le méthanol et en partie l'isophorone, de préférence au plus 10 %, étant séparés à la tête de la colonne,
et
b) l'isophorone-nitrile, et la quantité résiduelle d'isophorone et des impuretés (composants de point d'ébullition élevé) étant séparés dans le fond de la colonne ;
II. le courant de produits de I.b) est purifié dans une deuxième colonne de distillation et l'isophoronenitrile, l'isophorone et des impuretés sont séparées à partir du fond de la colonne ;
III. le mélange de II formé dans le fond est introduit dans une cristallisation à l'état fondu, les impuretés étant séparées et l'isophorone-nitrile étant isolé.
